# EUROPÄISCHE PATENTSCHRIFT

(11) **EP 3 577 453 B1**
(45) Veröffentlichungstag und Bekanntmachung des Hinweises auf die Patenterteilung: **12.01.2022**
(21) Anmeldenummer: 17784281.2
(22) Anmeldetag: 13.10.2017
(51) Int. Cl.: G01N 30/88, G01N 33/00

(54) **VERFAHREN ZUM KALIBRIEREN UND MESS- UND ANALYSEVERFAHREN**
METHOD FOR CALIBRATION, AND MEASUREMENT AND ANALYSIS METHOD
PROCÉDÉ D'ÉTALONNAGE ET PROCÉDÉ DE MESURE ET D'ANALYSE

(30) Priorität: 02.02.2017 DE 102017201680
(43) Veröffentlichungstag der Anmeldung: 11.12.2019
(73) Patentinhaber: Bentekk GmbH, 21073 Hamburg (DE)
(72) Erfinder: SCHMITTMANN, Matthias, 20146 Hamburg (DE); WEBER, Johannes, 20099 Hamburg (DE); WEBER, Paul, 13357 Berlin (DE); JÜNEMANN, Arne, 21075 Hamburg (DE)
(74) Vertreter: Glawe, Delfs, Moll
(86) Internationale Anmeldenummer: PCT/EP2017/076244
(87) Internationale Veröffentlichungsnummer: WO 2018/141427

(56) Entgegenhaltungen:
- EP-A1- 2 065 704
- DE-A1- 3 507 386
- US-A- 5 313 061
- US-A1- 2012 192 623
- US-A1- 2016 077 071

## Beschreibung

In Industrie, Arbeitssicherheit, Umweltschutz und Wissenschaft werden Gasmessgeräte eingesetzt, um Anwesenheit und Konzentration von chemischen Verbindungen in der Luft oder in abgeschlossenen Volumen zu bestimmen, oft mit dem Ziel, Spuren von umwelt- und gesundheitsschädlichen Stoffen nachzuweisen. Hierzu können entweder Proben für spätere Laboruntersuchungen genommen oder direkt Messungen mit Gasmessgeräten vor Ort durchgeführt werden. Üblicherweise bieten Labore eine höhere Sensitivität, Selektivität und Präzision der Messung, da die stationären Gasmessgeräte leistungsfähiger sind und die Bedingungen und Abläufe in der geschützten Laborumgebung durch geschultes Personal besser kontrolliert werden können. Durch die zunehmende Verfügbarkeit komplexerer Messgeräte, die gleichzeitig kompakt und transportabel sind, entstehen neue Herausforderungen für den Ablauf des gesamten Messverfahrens, um auch im Feldversuch präzise und wiederholbare Messungen zu ermöglichen, ohne dem Anwender durch umständliche Vorbereitung die Vorteile eines schnell einsetzbaren Gerätes zu nehmen. Diese Erfindung ermöglicht beispielsweise Personen des Arbeitsschutzes oder der Werkfeuerwehr Messgeräte mit hoher Leistungsfähigkeit einzusetzen, die bisher ausschließlich hochqualifizierten Personen aus der Laboranalytik vorenthalten war.

Im Kontext der vorliegenden Patentanmeldung werden unter Gasmessgeräten solche Messgeräte verstanden werden, die die chemische Zusammensetzung und Konzentration von Gasen bestimmen, nicht solche die primär andere Eigenschaften (z.B. Temperatur) von Gasen messen. Verschiedene Gasmessgeräte unterscheiden sich im Aufbau und in den verschiedenen Detektionsmethoden, weniger im Verfahren der Anwendung. Ein voller Verfahrenszyklus beim Anwender umfasst zunächst eine Vorbereitung (Kalibrierung und oft Spülung) des Gasmessgerätes, dann die Messung an einer Probe und anschließende Auswertung. Die Probe wird entweder direkt gasförmig in das Gasmessgerät gegeben oder in einem Ofen bzw. einer Anreicherungseinheit verdampft. Bei der Messung wird ein Signal aufgezeichnet, das mit den Signalhöhen vorheriger Messungen verglichen wird, im einfachsten Fall einer Normierungsmessung, die einmalig vor Auslieferung durch den Hersteller oder regelmäßig durch den Anwender durchgeführt wird. Alternativ oder ergänzend wird das Messsignal auch mit Erwartungswerten der Signalhöhe aus theoretischen Modellen verglichen oder verrechnet. Die Analyse erfordert immer den Abgleich der Probenmessung mit einer experimentell bestimmten oder theoretischen Referenz. Vor allem im Feldversuch liegen von Messung zu Messung unterschiedliche Bedingungen vor, die in äußere Faktoren (Temperatur, Luftdruck, Luftfeuchtigkeit) und in innere Faktoren (Leistungsschwankungen, Verschmutzung, Alterung und Abnutzung von Bauteilen des Gasmessgerätes) unterschieden werden können. Um eine verlässliche Analyse auszuführen, muss die Abweichung der veränderlichen Bedingungen während der Messungen korrigiert werden. Um das Messsignal am Detektor und ein theoretisches Modell zu vergleichen, müssen diese über eine mathematische Funktion verknüpft werden, die sogenannte Gerätefunktion, in der die Information über das Gasmessgerät enthalten ist.

Eleganter sind Messverfahren, die auf ein absolutes Messergebnis verzichten und stattdessen die Auswertung aufgrund einer relativen Messung vornehmen. Eine solche relative Methode ist die Verbindung der Messung an einer unbekannten Probe mit einer Messung an einer bekannten Referenz. Eine solche Referenz-Kalibrierung wird üblicherweise vor der Probenmessung ausgeführt. Als Referenz eignet sich ein Gasgemisch mit bekannter chemischer Zusammensetzung und Konzentration. Dieses kann direkt gasförmig in einem Behälter vorgehalten werden, wobei das Gemisch in dem Behälter höher konzentriert und komprimiert sein kann und erst für die Kalibrierung in einem Trägergas verdünnt wird. Alternativ können die Referenzstoffe im flüssigen Zustand vorgehalten werden, wobei diese flüssigen Referenzstoffe für die Kalibrierung in einem Ofen verdampft werden oder dauerhaft durch die Membran eines Permeationsröhrchens diffundieren. Zusätzlich zu einer Kalibriermessung mit dem Hauptdetektor können auch die Messungen von Hilfssensoren, z.B. Thermometer, oder im Gasmessgerät erzeugte oder gespeicherte Daten in die Korrektur einbezogen werden.

Auch bei einfachen Gasmessgeräten, die eine unmittelbare Messung mit einem einzigen Detektor ohne Trennung der Stoffe durchführen, ist es angebracht oder sogar vorgeschrieben, zumindest an jedem Tag, an dem das Gerät eingesetzt wird, eine Kalibrierung vorzunehmen. Neue Kalibrierungen sind in der Regel erforderlich, wenn sich die äußeren Bedingungen, etwa Temperatur, Feuchtigkeit und Luftdruck verändern, da sich diese auf die Stoffe, den Messvorgang und damit die Ergebnisse auswirken können. Eine Kalibrierung an einer bekannten Referenzprobe erlaubt eine systematische Abweichung festzustellen und zu korrigieren. Eine wiederholte Messung an einer bekannten, unveränderten Referenzprobe ermöglicht Schlüsse über die Stabilität des Messverfahrens und systematische Veränderungen durch Alterung des Gerätes. Bei einfachen Detektoren mit eindimensionaler Messgröße entspricht die Kalibrierung oft einer Normierung der Messung um einen bei der Kalibrierung zu bestimmenden Faktor. Zusätzliche Herausforderungen ergeben sich für komplexere Messverfahren, bei denen eine mehrdimensionale Messung, etwa ein Gaschromatogramm, aufgezeichnet und ausgewertet werden, da eine ordentliche Kalibrierung in diesen Fällen eine Korrektur über eine entsprechende Funktion erfordert, also etwa die Koeffizienten einer polynomischen Korrekturfunktion bestimmt werden müssen. Die Durchführung einer solchen Berechnung ist nicht jedem Anwender möglich und verzögert die Anwendung im Feld, wodurch ein wichtiger Vorteil gegenüber Laborverfahren, nämlich die kurzfristige Verfügbarkeit von Messergebnissen, verringert wird.

Ein erster Stand der Technik sind portable einfache Gasmessgeräte mit direkter Anzeige der Messergebnisse (sogenannte Skalenanzeige, engl. direct-reading measurement) entweder für die kontinuierliche oder punktweise Messung, die mit einer großen Auswahl von Detektoren und Konfigurationen verfügbar sind, die für die jeweiligen Zielstoffe und Spezifikationen geeignet sind. Diesen Geräten ist gemeinsam, dass sie jeweils für einen bestimmten Stoff oder eine Gruppe von Stoffen ein eindimensionales Signal bzw. eine Zeitreihe eindimensionaler Signale aufzeichnen können (unter "Dimension" wird vorliegend die Anzahl unterschiedlicher Stoffe/Stoffgruppen verstanden, also bezieht sich "eindimensional" auf nur einen einzigen Stoff bzw. Stoffgruppe). Bei älteren Geräten wird als Messwert ein unverarbeitetes Detektorsignal angezeigt, das der Anwender dann selbst umrechnen muss, um die Konzentration des Zielstoffes zu bestimmen. In vielen aktuellen Ausführungen wird diese Umrechnung bereits durch eine integrierte Recheneinheit des Gasmessgerätes durchgeführt und das Detektorsignal einer Konzentration des detektierten Stoffes zugeordnet. Auch die Daten aus der Kalibriermessung werden bei diesen Geräten automatisch verarbeitet und sind im angezeigten Messwert bereits verrechnet. Diese direkte Ablesbarkeit der für den Anwender relevanten Information in Verbindung mit einer einfachen Bedienung dieser Geräte ermöglicht einen zuverlässigen Einsatz durch Anwender ohne wissenschaftliche Ausbildung. Die Auswahl und Durchführung der Verfahrensschritte von Spülung, Kalibrierung und Messung erfordern keine komplizierten Eingaben. Auch deshalb sind diese Gasmessgeräte mit eindimensionaler Messgröße als portable Messgeräte für Feldversuche weit verbreitet. Aus der US 2016/077071 A1 ist ein portables Messgerät mit einer werksseitigen Kalibrierung bekannt, das eine gemessene Konzentration eines Zielgases selbsttätig korrigiert anhand der tatsächlichen Luftdichte. Ein Funktionstest kann teilautomatisiert erfolgen. Es sind Datenbanken für Kalibrierungs- und Instrumentenkonfigurationswerte vorgesehen. Ermittelte Daten können in einer zentralen Datenbank abgelegt werden. Die einstufige Kalibrierung erfolgt anhand eines Zielstoffs. Zum großen Nachteil dieser Geräte lässt sich mit einer eindimensionalen Messgröße keine Auswertung vornehmen, deren Ergebnis die jeweiligen Konzentrationen mehrerer Stoffe aufgetrennt bestimmt oder zumindest einzelne Stoffe identifiziert. Bei einer Mehrzahl von Stoffen, für die der Detektor sensitiv ist, kann nur das Summensignal bestimmt werden. Außerdem kommt es zu vielen falschpositiven Messungen, bei denen ein hohes Signal gemessen wird, das durch irrelevante Stoffe (z.B. Wasserdampf) entsteht. Oft werden solche Messgeräte mit direkter Anzeige nur zur Hilfe verwendet, um anschließend Proben zu nehmen, die in einem Labor ausgewertet werden.

Ein zweiter Stand der Technik sind Laborgeräte für die Messung von Gasen, die für die Anwendung durch Wissenschaftler oder andere geschulte Fachkräfte konzipiert sind. Verbreitete Laborgeräte sind Gaschromatographen, Massenspektrometer, optische Spektrometer oder Kombinationen dieser Geräteklassen. Bei solchen Geräten wird für jeden Messpunkt ein mehrdimensionales Messergebnis aufgezeichnet: Bei Gaschromatographen (GC) wird ein Chromatogramm aufgezeichnet, bei dem die Intensität an einem Detektor gegen die Retentionszeit in einer Chromatographie-Säule aufgetragen wird. Bei Massenspektrometern (MS) wird ein Massenspektrogramm aufgezeichnet, in dem die Häufigkeit von Stoffen in Abhängigkeit ihres Masse-Ladungs-Verhältnisses aufgetragen wird. Bei optischen Spektrometern wird die Transmission, Absorption oder Reflektion von Licht bei verschiedenen Wellenlängen in bzw. an den Gasen gemessen. Bei höherdimensionalen Verfahren, z.B. dem GC-MS, erhält man entsprechend mehrdimensionale Messergebnisse. Bei kontinuierlich oder regelmäßig aneinandergereihten Messpunkten kann die Messung zusätzlich als Zeitreihe notiert werden, was aber nicht als zusätzliche Dimension der Messung zu verstehen ist, da jeder Messpunkt in der Zeitreihe einzeln ausgewertet wird. Die mehrdimensionale Messung erlaubt eine Trennung von einzelnen Bestandteilen der Signale, die verschiedenen Stoffen zugeordnet werden. Hier ist anzumerken, dass etwa bei der optischen Spektroskopie die Stoffe nicht physisch getrennt werden, sondern nur über die charakteristischen Messwerte identifiziert werden können. Die Auswertung dieser Signale erfolgt heute meist unterstützt oder sogar automatisiert in einem Computerprogramm. Der Vorteil von Laborgeräten ist deren hohe Leistungsfähigkeit, die sich durch ein kompromisslos konzipiertes Gerät ergibt. Nachteil von Laborgeräten ist dagegen der hohe Zeit- und Arbeitsaufwand durch eine Fachkraft. Für den Einsatz im Feldversuch sind diese Geräte nicht geeignet, bestenfalls installiert in einem Messfahrzeug.

Ein dritter Stand der Technik sind miniaturisierte Laborgeräte für den mobilen Einsatz, die eine stoffspezifische Konzentrationsmessung erlauben und hierfür für jeden Messpunkt eine mehrdimensionale Messgröße aufzeichnen. Auch in dieser Klasse gibt es Geräte mit unterschiedlichen Detektoren (z.B. GC, MS, Spektrometer). Eine Verbindung der einfachen Anwendung des ersten Stands der Technik für mobile Gasmessgeräte mit der hohen Präzision und Zuverlässigkeit des zweiten Stands der Technik für Laborgasmessgeräte wird dabei nicht erreicht. Frühe Versuche, stofftrennende Laborverfahren in tragbaren Gasmessgeräten umzusetzen, resultierten oft in unhandlichen schweren Geräten, die zusätzlich einen Laptop-Computer zur Ansteuerung erforderten. Aber auch wenn die Technologie erfolgreich miniaturisiert wird, übernehmen viele Hersteller das Verfahren oft unverändert von Laborgeräten mit entsprechenden Anforderungen an Zeit und Kenntnisse der Anwender. Das ist unbefriedigend, so dass sich derartige Geräte nicht breit durchsetzen konnten. Von der Anmelderin vorgeschlagen (DE 10 2015 219 838) sind moderne miniaturisierte Gasmessgeräte, die mit den Möglichkeiten der sogenannten Digitalisierung entwickelt wurden. Genutzt werden handelsübliche und kostengünstige Recheneinheiten und digitale Datenverbindungen, wie sie in digitalen Mobilgeräten massenhaft verfügbar sind, um so in der Gasmesstechnik auf effektiv unbegrenzte Datenspeicher und leistungsfähige Recheneinheiten zuzugreifen, die im virtuellen Teil des Verfahrens entweder von der Recheneinheit des Gasmessgerätes, einem verbundenen Mobilcomputer oder sogar durch einen räumlich getrennten Server, der über eine Netzwerkverbindung an das Gasmessgerät verbunden ist, zur Verfügung gestellt werden. Diese Leistungsfähigkeit ermöglicht die schnelle Ausführung von komplexen Messprogrammen und Auswertungsalgorithmen und auch eine zugänglichere Bedienung und Anzeige des Gasmessgerätes.

Trotz dieser Modernität erfordern auch diese Gasmessgeräte eine Kalibrierung vom Anwender. Dies geschieht in der Weise, dass mit dem Gasmessgerät durch den Anwender regelmäßig eine Kalibrierung in Form einer Messung an einem Gasstandard bekannter chemischer Zusammensetzung und Konzentration vorgenommen wird. Ein tragbares Gerät im Kofferformat zur Gasanalyse ist offenbart in der EP 2 065 704 A1. Es weist einen miniaturisierten Gaschromatograph auf mit einer Mikroprozessor-Elektronik als Steuer- und Auswerteeinheit. Über sie werden im Betrieb die Kalibrierung und die Messzyklen gesteuert. Es weist zwei Probegasanschlüsse auf, wobei unter der Steuerung der Mikroprozessor-Elektronik eine Parametrierung und Kalibrierung erfolgen kann. Ein Messbetrieb umfasst Aufzeichnen der Signale von dem Gaschromatograph und automatisierte Auswertung. Unter Nutzung der Mikroprozessor-Elektronik realisierbar sind u.a. Kalibrierung, Rekalibrierung und Selbstvalidierung. Die Erfahrung zeigt, dass viele Anwender ein Verfahren einmalig einstudieren und dieses wiederholen. Die Lernkurve zur fortgeschrittenen Bedienung der Gasmesseräte ist vergleichsweise flach, vor allem weil die Anwender häufig einen sehr hohen Wissensstand und Erfahrung in der Anwendung von Gasmesstechnik besitzen. Dabei schätzen auch erfahrene Anwender die vereinfachte Bedienung und Zeitersparnis dank einer schnellen Messung. Eine Anpassung der Parameter an veränderte Bedingungen unterbleibt häufig. Es besteht also die Situation, dass für eine einfache, eindimensionale Messung die Kalibrierung leicht gelingt und durch die Anwender auch vorgenommen wird. Bei komplexen, mehrdimensionalen Messungen ist die Kalibrierung jedoch zu kompliziert.

Es ist die Aufgabe der Erfindung, die Vorteile der einfachen Kalibrierung auch für die mehrdimensionale Messung eines entsprechend komplexen Gasmessgeräts nutzbar zu machen.

Die erfindungsgemäße Lösung liegt in den Merkmalen der unabhängigen Ansprüche. Vorteilhafte Weiterbildungen sind Gegenstand der abhängigen Ansprüche.

Bei einem Verfahren zum mehrstufigen Kalibrieren von Mess- und Analyseverfahren für tragbare Gasmessgeräte mit verbundener Steuereinheit und verbundener Auswerteeinheit mit einer Detektoreinheit für Gaschromatographie, zur selektiven Detektion einer Mehrzahl von chemischen Verbindungen, wobei in einem ersten Schritt nach Herstellung eine Werkseinstellung des Gasmessgeräts vorgenommen wird, wobei eine Datenbank zu einer Mehrzahl von Zielstoffen und/oder Messprogrammen für das jeweilige Gasmessgerät generiert wird, in einem zweiten Schritt durch den Anwender ein Behälter mit einem bekannten Gasstandard an das Gasmessgerät angeschlossen wird und an diesem Gasstandard eine Referenzmessung durchgeführt wird, bei der eine mehrdimensionale Messgröße, insbesondere ein Gaschromatogramm, aufgezeichnet wird, in einem dritten Schritt durch den Anwender ein Messprogramm für einen oder mehrere Zielstoffe aus einer Liste ausgewählt wird, die durch den Hersteller auf das Gasmessgerät aufgespielt wurden oder durch den Anwender erstellt sein können, in einem vierten Schritt die Messung ausgelöst wird, wobei die Steuereinheit das Messverfahren in Abhängigkeit des gewählten Messprogramms ausführt, bei dem eine mehrdimensionale Messgröße aufgezeichnet wird, und in einem fünften Schritt die Auswerteeinheit automatisiert eine Analyse zur Identifizierung der in der Probe vorhandenen Zielstoffe und deren jeweilige Konzentrationen durchführt, wobei ein Algorithmus mehrere Eingabegrößen verarbeitet, darunter die mehrdimensionale Messgröße aus der Messung an der Probe, die mehrdimensionale Referenzmessung am Gasstandard, den Werten für den Zielstoff oder die Zielstoffe für das jeweilige Programm und die Werkseinstellung für das Gasmessgerät.

Zuerst seien einige verwendete Begriffe erläutert: Unter einem Herstellen wird die eigentliche Erstherstellung (Produktion) verstanden, aber auch eine Wiederherstellung im Sinne einer Reparatur.

Unter selektiver Detektion wird verstanden, dass die Auswertung der mehrdimensionalen Messgröße eine Unterscheidung zwischen den Signal-Anteilen von verschiedenen Zielstoffen und eventuellen Störsignalen in der Messung ermöglicht und damit für einen oder mehrere Zielstoffe, deren Anwesenheit in der Probe nicht bekannt sein muss, die Berechnung von Anwesenheit und Konzentration erlaubt. Beispielsweise wird für ein unbekanntes Probengas eine Konzentration K_{X} für Stoff X und Konzentration K_{Y} für Stoff Y ermittelt, wobei weitere Bestandteile des Signals als Hintergrund oder Störsignale identifiziert werden. Im Gegensatz kann bei der nicht-selektiven Detektion nicht zwischen verschiedenen für die Detektoreinheit messbaren Stoffen, beispielsweise einem gefährlichen Zielstoff, einem ungefährlichen weiteren Stoff und/oder Störsignalen unterschieden werden, weshalb es zu nicht-eindeutigen oder falschpositiven Messergebnissen kommen kann. Auch eine grundsätzlich selektive Detektion kann erst ab einer gewissen Signalhöhe zuverlässig funktionieren, weshalb auch die ausreichende Sensitivität der Detektoreinheit für alle in der Probe vorhandenen Stoffe bzw. deren Konzentration für die Selektivität erforderlich ist. Für eine automatisierte Analyse einfach zu lösen sind solche Fälle, in denen alle Bestandteile des Signals deutlich getrennt sind, etwa als nicht-überlappende Signalspitzen, die mit einem konstanten Hintergrundsignal und geringem Rauschen überlagert sind. Eine Herausforderung für einen Analyse-Algorithmus sind solche Fälle, bei denen die SignalCharakteristiken mehrerer Zielstoffe für eine bestimmte Detektoreinheit sich ähnlich sind oder die Messsignale mehrerer Zielstoffe nicht unabhängig sind, also eine sog. Kreuzsensitivität vorliegt, wodurch trotz ausreichender Sensitivität für die einzelnen Stoffe keine Selektivität der Detektoreinheit für diese Messung gegeben ist.

Unter einer abgesetzten Datenbank wird eine Datenbank verstanden, die räumlich entfernt in einer anderen Geräteeinheit angeordnet ist.

Unter einem Programm wird vorliegend ein gesteuerter Ablauf einer Messung verstanden ("Messprogramm").

Unter gesundheitsschädlichen Stoffen werden solche chemischen Verbindungen verstanden, die für den Menschen direkt toxisch wirken oder sich durch Langzeitfolgen negativ auf die Gesundheit auswirken, insbesondere das Risiko für Krebserkrankungen erhöhen. Je nach Rechts- und Anwendungsbereich gelten unterschiedliche gesetzliche oder sonstige Regeln zur Festlegung der Grenzkonzentration von Gefahrenstoffen. Im Bereich der Arbeitssicherheit in Deutschland sind die Gefahrenstoffe in den technischen Regeln TRGS 900 und TRGS 910 mit den jeweiligen Arbeitsplatzgrenzwerten, Akzeptanz- bzw. Toleranzkonzentrationen festgeschrieben, diese werden gegebenenfalls durch betriebliche Regelungen ergänzt. Konkrete Beispiele für Gefahrenstoffe in Bezug auf die Erfindung sind insbesondere Benzol, 1,3-Butadien, Trichlorethen, Ethylenoxid und Toluol (letzteres ab einer Konzentration von 50 ppm). Als Referenzstoffe für eine Kalibriermessung für diese Gruppe eignen sich die nach den technischen Regeln ungefährlichen Stoffe Isobuten und Toluol (jeweils in einer Konzentration von 10 ppm). Am Beispiel Toluol wird deutlich, dass je nach Konzentration die Stoffe als gesundheitsschädliche Zielstoffe oder ungefährliche Kalibrierstoffe für das Verfahren von Bedeutung sein können.

Der Kern der Erfindung besteht darin, ein mehrstufiges Kalibrierverfahren zu schaffen, welches eine Einstellung (bzw. Kalibrierung) ab Werk verknüpft mit einer Kalibrierung durch den Anwender, in Verbindung mit einer unterstützenden Automatisierung, die vom Anwender im Einsatz ausgeführte Mess- und Analysevorgänge anpasst und ggf. korrigiert. Hierbei bedingen beide Teile einander.

In einem ersten Teil des Verfahrens wird dabei das Gasmessgerät durch den Hersteller umfassend eingestellt und vorbereitete Messprogramme aufgespielt und Testmessungen durchgeführt. Diese Daten sind Teil der Datenbank, auf die der Algorithmus zur Auswertung zugreifen kann. Dieser erste Teil wird nach der Herstellung und nach Wartungen sowie Reparaturen durchgeführt, bevor das Gasmessgerät ausgeliefert wird. Erst der zweite Teil des Verfahrens wird durch den Anwender als Routine ausgeführt. Bei der Kalibrierung durch den Anwender werden eine (oder mehrere) Referenzmessungen an einem bekannten Gasstandard vorgenommen, der keinen Zielstoff enthalten muss. Vor der Messung wählt der Anwender ein Messprogramm aus, das für einen oder eine Gruppe von Zielstoffen optimiert ist. Eventuell bietet das Gasmessgerät dem Anwender zusätzliche Optionen an, die für das jeweilige Programm verfügbar sind. Nun wird die Messung durchgeführt, wobei die Parameter der Messung mit Informationen aus der gespeicherten Datenbank angepasst werden. Bei der Auswertung werden die Informationen aus Probenmessung, Kalibrierung und der werkseitig installierten Datenbank zu Programmen, Stoffen und Kalibrierung ohne weitere Eingaben des Anwenders verarbeitet. Sofort werden die gemessenen Konzentrationen der identifizierten Stoffe in der Probe angezeigt.

Das erfindungsgemäße Verfahren ermöglicht eine Kombination von dem geringen Aufwand einer Programmauswahl und einfachen Kalibrierung an einem Gasstandard, wie an sich für einfache Handmessgeräte üblich, mit einem komplexen computergestützten Mess- und Analyseprogramm, wie es bisher nur wissenschaftlichem Personal mit Laborgeräten in Laborumgebung möglich ist. Möglich wird ein solches neues Verfahren durch die weitergeführte Entwicklung von Miniaturisierungen von stoffspezifischen transportablen Gasmessgeräten, die sich technologisch so weit von den Laborgeräten entfernt haben, dass sie nach eigenen Verfahren verlangen. Praxistauglich wird ein solches Verfahren durch die Verfügbarkeit von hoher Rechenleistung und großer Datenspeicher in digitalen Mobilgeräten, die eine automatisierte Ausführung anspruchsvoller Prozess- und Datenoperationen für Kalibrierung, Messprogramme und Auswertung ermöglicht und dem Anwender eine zugängliche Bedienungsoberfläche bietet. In diesem Verfahren gleicht der zweite Verfahrensteil der durch den Anwender vorgenommenen Kalibrierung dem bei den verbreiteten einfachen Messgeräten üblichen Verfahren, weshalb die Ausführung dieser Verfahrensschritte solchen Anwendern bekannt ist, die bisher ein nicht-spezifisches Gerät verwendet haben. Eine bessere Anpassung des Mess- und Analyseverfahrens an äußere Faktoren erlaubt die zuverlässige Anwendung in wechselnden Umweltbedingungen, während die Anpassung an veränderte innere Faktoren die Zeiträume verlängern, bevor Teile ausgetauscht werden müssen. Die Aufzeichnung aller Kalibriermessungen als Reihe erlaubt Schlüsse über den Zustand des Gasmessgerätes und die Notwendigkeit eines Austauschs von Teilen des Gasmessgerätes.

Zweckmäßigerweise wird durch den Hersteller eine Werkskalibrierung durchgeführt, bei der für das jeweilige Gasmessgerät eigene Gerätekorrekturwerte ermittelt und in einer Datenbank gespeichert werden. Mit der Werkskalibrierung kann eine bessere Berücksichtigung geräteindividueller Besonderheiten erreicht werden, wodurch sich die Messgenauigkeiten erhöhen.

Mit Vorteil handelt es sich bei dem Gasmessgerät um einen Gaschromatographen, der als miniaturisiertes, tragbares Messgerät für Feldversuche unter 1,5 kg Gesamtgewicht ausgeführt ist.

Zweckmäßigerweise ist weiter das Gasmessgerät als miniaturisiertes, tragbares Messgerät für Feldversuche ausgeführt. Dank der Miniaturisierung ist eine hohe Portabilität gewährleistet, wodurch sich das Einsatzspektrum erweitert.

Vorzugsweise wird bei der Kalibriermessung als Gasstandard ein Gasgemisch der Stoffe Isobuten (A) und Toluol (B) in jeweils ungefährlicher Konzentration, insbesondere jeweiligen Konzentrationen zwischen 1 ppm und 100 ppm in Luft oder Stickstoff, verwendet, wobei die Zielstoffe des Messprogramms der Probenmessung die bereits in geringer Konzentration schädlichen Stoffe Benzol (X) und 1,3-Butadien (Y) sind, deren Detektionsgrenze jeweils unter 1 ppm, vorteilhaft sogar unter 1 ppb, liegt. Somit können in der Analyse auch Signalanteile weiterer unbekannter Stoffe (Z) im Messsignal von den Signalanteilen der Zielstoffe (X, Y) unterschieden werden.

Mit Vorteil ist das tragbare Gasmessgerät mit einer mobilen Recheneinheit ausgestattet oder verbunden, die über eine drahtlose Verbindung auf Datenbanken von externen Recheneinheiten zugreifen kann. Dadurch kann das eigentliche Gasmessgerät noch kleiner und damit noch portabler ausgeführt werden. Ferner hat der Zugriff auf externe Datenbanken/Recheneinheiten den Vorteil, dass umfangreichere Analysefunktionen auf der Recheneinheit ausgeführt werden können, und zwar auch unter Rückgriff auf ggf. externe Datenbanken. Es kann so auf eine Art Expertensystem zurückgegriffen werden, wodurch sich die Qualität der Messung weiter steigern lässt. Letzteres gilt vor allem dann, wenn das Gasmessgerät zusätzlich Daten aus externen Datenbanken abruft, die durch den Algorithmus der Analyse verarbeitet werden.

Besonders bewährt hat sich für die Erfindung, wenn das Gasmessgerät die mehrdimensionale Messgröße aufeinanderfolgender Messungen in einer Datenbank speichert und dabei über einen Algorithmus aus den Messergebnissen der Referenzprobe einen Funktionstest des Gasmessgerätes durchführt, nachdem das Gasmessgerät dem Anwender Informationen über den Zustand des Gasmessgerätes, insbesondere eine Warnung über eine nötigen Wartung, anzeigt. Die Betriebssicherheit und die Robustheit der Messungen kann so gesteigert werden. Ferner können auch evtl. schleichende Verschlechterungen, beispielsweise der Detektoreinheit, frühzeitig erkannt und eine Reparatur veranlasst werden, bevor eine Verschlechterung der Messgüte eintritt. Damit wird die Analyse- und Messqualität weiter gesteigert.

In entsprechender Anwendung kann zur Verbesserung des laufenden Messbetriebs vorgesehen sein, dass das Gasmessgerät vorangegangene Messergebnisse, die ebenfalls mit dem gewählten Programm erzeugt wurden, aus einer internen Datenbank in den Algorithmus der Analyse einbezieht. Alternativ oder zusätzlich kann das Gasmessgerät Messergebnisse, die von anderen Gasmessgeräten ebenfalls mit dem gewählten Programm erzeugt wurden, aus einer internen oder externen Datenbank in den Algorithmus der Analyse einbeziehen. Auf diese Weise kann durch Abgleich mit früheren eigenen Messungen und/oder den Messungen anderer Messgeräte, die insbesondere in der externen Datenbank gespeichert sind, eine Verbesserung des Auswerte-Algorithmuses der Analyse erreicht werden. Insbesondere können so Filter bei der Signalverarbeitung besser parametriert werden und eventuelle Modellfehler verringert werden. Insgesamt kann so eine beträchtliche Steigerung der Ergebnisgüte erreicht werden.

Mit Vorteil wird das Messverfahren automatisiert an die Messergebnisse von Hilfsdetektoren, insbesondere der Außentemperatur, angepasst. Es lässt sich so prüfen, ob die Messungen mit unveränderter Qualität weitergeführt werden können oder ob zur Erhaltung eine erneute Kalibrierung durch den Anwender erforderlich wird.

Gemäß einem besonders vorteilhaften Aspekt der Erfindung, der gegebenenfalls unabhängigen Schutz verdient, schlägt das Gasmessgerät dem Anwender automatisiert Änderungen am Messprogramm vor, die es aus den in Datenbanken gespeicherten vorangegangenen Messungen in einem Algorithmus als mit Wahrscheinlichkeit geeignetere Parameter berechnet hat. Auf diese Weise kann unter Nutzung von Vorwissen eine Steigerung der Analysequalität erreicht werden. Alternativ oder zusätzlich kann vorgesehen sein, dass der Algorithmus der Analyse, nachdem ein vorangegangener Durchgang der Analyse nach bestimmten Kriterien als fehlgeschlagen bewertet wird, automatisiert nachträglich weitere Zielstoffe aus einer Datenbank in das Programm der Analyse hinzufügt, die das durch den Anwender vorgewählte Messprogramm ergänzen, wobei dieser Vorgang wiederholt wird bis entweder die Analyse erfolgreich wird oder eine bestimmte Zahl von Wiederholungen bzw. Zeitspanne überschritten wird. Es wird so eine iterative Verfahrensführung erreicht, die bei konvergentem Verhalten eine höhere Analysequalität erreichen kann und bei divergentem Verhalten entsprechend reagiert, zum Beispiel durch Wiederholung oder Abbruch. Unnötige Verzögerungen werden so vermieden, und dennoch wird eine beträchtliche Steigerung der Analysequalität erreicht.

Ferner kann vorgesehen sein, dass die Messung parallel von mehreren Anwendern durchgeführt wird, und während des Messeinsatzes die Einstellung der Messprogramme und/oder Daten zwischen mehreren in Netzwerken verbundenen Gasmessgeräten und/oder zu einem zentralen Server übermittelt werden.

In einer vorteilhaften Ausführung wird die Kalibrierung mit einem Gasstandard durchgeführt, der am Gasmessgerät oder im Gasmessgerät vorgehalten wird, möglicherweise auch parallel während der Probenmessung. Durch diesen weiteren Schritt wird das manuelle Anschließen eines Gasstandards oder sogar der gesamte Verfahrensschritt der Kalibrierung durch den Anwender eingespart, da die Kalibrierung während einer (oder während jeder) normalen Messung durchgeführt wird. Vorteilhaft sollte die Möglichkeit bestehen, wahlweise nur die Kalibrierung ohne Probe durchzuführen oder den Gasstandard nicht mit in die Probe zu mischen, um mögliche Probleme mit Kreuzempfindlichkeiten und überlappenden Signalen von Kalibrierung und Probe in einer Messung vermeiden zu können. Der Gasstandard kann flüssig in Permeationsröhrchen oder gasförmig komprimiert in Kartuschen innerhalb oder am Gasmessgerät vorgehalten werden. Wird der verwendete Gasstandard parallel zur Probenmessung in die Detektoreinheit injiziert (=interner Standard) darf keiner der Zielstoffe darin enthalten sein und muss eine ausreichend abweichende Charakteristik zur erwarteten Charakteristik der Zielstoffe haben, um sauber vom Signal der Probe getrennt werden zu können. Wird die Kalibrierung durch den Anwender zwischen Messungen durchgeführt, ist es hingegen vorteilhaft, ähnliche oder identische Stoffe als Gasstandard zu wählen.

Zweckmäßigerweise ist eine automatische Erkennung und Vermeidung von Querempfindlichkeiten vorgesehen. Dazu werden in einem Messprogramm zur Messung mehrerer Zielstoffe (oder einer Auswahl einer Gruppe von Zielstoffen aus einer Liste von Stoffen) die Querempfindlichkeiten ermittelt bzw. abgerufen, und bei relevanten Querempfindlichkeiten wird das Steuerprogramm der Messung und/oder der Algorithmus der Analyse an der Auswerteeinheit automatisch so angepasst, dass eine bekannte Querempfindlichkeit für diese Zielstoffe vermieden wird. Der Anwender wird damit von dieser schwierigen und ggf. sehr fehleranfälligen Aufgabe entlastet, was der Gefahr von unerkannten (ggf. gravierenden) Fehlmessungen entgegenwirkt. Vorzugsweise wird das tragbare Gasmessgerät als Teil oder Aufsatz an einem durch den Anwender ferngesteuerten und/oder selbstgesteuerten Fahrzeug und/oder Fluggerät, insbesondere einer Roboter-Drohne, als Trägereinheit verwendet, wobei vorzugsweise das Gasmessgerät über eine drahtlose Verbindung mit einer externen Recheneinheit verbunden wird.

Die Erfindung wird nachfolgend unter Bezugnahme auf die beigefügte Zeichnung erläutert, in der vorteilhafte Ausführungsbeispiele dargestellt sind. Es zeigen:
- Fig. 1: eine Übersichtsdarstellung eines Gasmessgeräts gemäß einem ersten Ausführungsbeispiel der Erfindung angeschlossen an ein Testgas (Gasstandard);

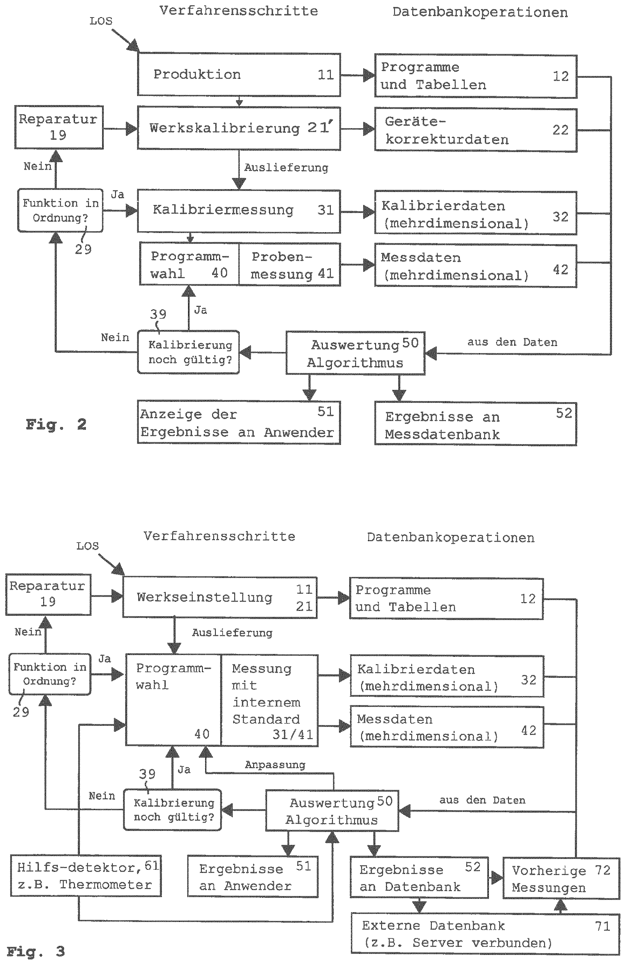

- Fig. 2: einen Verfahrensablauf gemäß einem ersten Ausführungsbeispiel;
- Fig. 3: einen Verfahrensablauf gemäß einem zweiten Ausführungsbeispiel;
- Fig. 4: ein schematisches Gaschromatogramm eines mehrdimensionalen Standards sowie einer entsprechenden Probe;
- Fig. 5a, b: Verfahrensabläufe zur iterativen Anpassung;
- Fig. 6a: ein Gasmessgerät gemäß einem weiteren Ausführungsbeispiel, mit integrierter Detektoreinheit, Bedien- und Anzeigeeinheit, sowie Steuer- und Analyseeinheit mit Datenbank;
- Fig. 6b: eine Variante mit einem Verbund von Gasmessgeräten, die über Netzwerkverbindungen gekoppelt sind; und
- Fig. 6c: einen Verbund mehrerer Gasmessgeräte und Anwender.

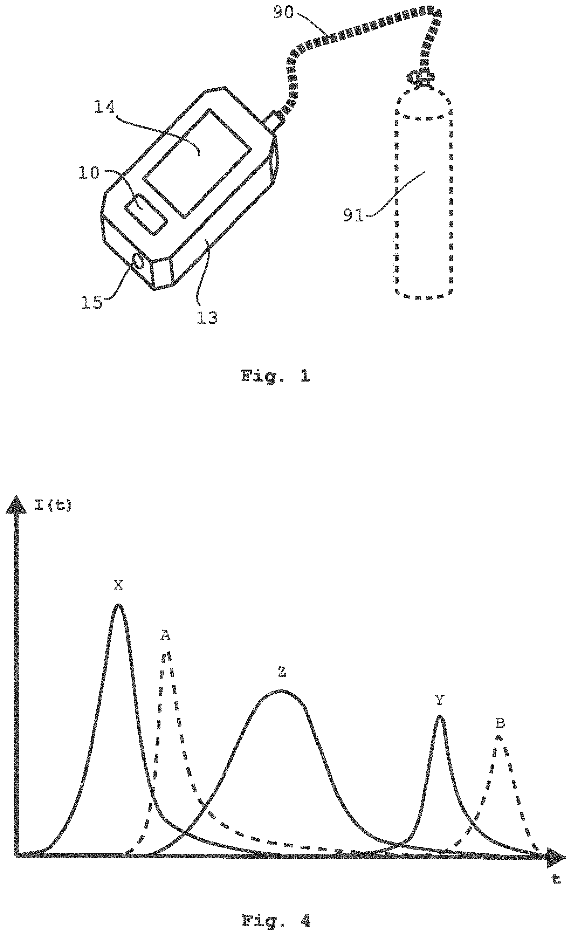

Ein tragbares Gasmessgerät 1 als tragbare Gasanalysevorrichtung mit einer daran über einen Verbindungsschlauch 90 angeschlossenen Gasflasche 91 ist in Fig. 1 dargestellt. Das tragbare Gasmessgerät 1 umfasst ein Gehäuse 13, an dessen Stirnseite ein Einlass 15 für eine Gaszuführung angeordnet ist. Auf einer Oberseite des Gehäuses 13 sind eine Anzeigeeinrichtung 14 sowie ein Bedienorgan 10 angeordnet. Ferner sind im Inneren des Gehäuses 13 eine Messeinrichtung, eine Recheneinrichtung sowie ein elektrischer Energiespeicher (nicht dargestellt) angeordnet. Es sei angemerkt, dass die Recheneinrichtung und/oder Anzeigeeinrichtung 14 optional auch extern angeordnet sein können, also außerhalb des Gehäuses 13. Es handelt sich dann um eine abgesetzte Rechen- bzw. Anzeigeeinrichtung. Zur Verbindung sind an sich übliche Übertragungsmittel vorgesehen, insbesondere Kurzstreckenfunk (über Bluetooth oder WLAN). In der Gasflasche 91 ist ein Gasgemisch mit definierter Zusammensetzung (Gasstandard 9) enthalten. Es enthält ein Trägergas und eine weitere Komponente und vorzugsweise noch weitere Komponenten (eindimensionaler bzw. mehrdimensionaler Gasstandard). Grundsätzlich sieht das erfindungsgemäße Verfahren eine Kalibrierung vor Aufnahme des Messbetriebs vor. Damit wird die tragbare Gasanalysevorrichtung 1 auf die jeweiligen Messanforderungen und Messumgebung eingestellt. Ein erster Teil davon braucht in der Regel nur einmal durchgeführt zu werden, nämlich im Anschluss an Produktion 11 der tragbaren Gasanalysevorrichtung 1, wobei auch verschiedene, in der Regel stoffspezifische Programme und Tabellen 4 auf die tragbare Gasanalysevorrichtung 1 aufgespielt werden. In der Regel wird dies noch im oder unter Verantwortung des Herstellerwerks erfolgen. Dabei kann es sich um eine Werkseinstellung 21 im Rahmen der Qualitätsprüfung des Herstellers oder um eine aufwendigere Werkskalibrierung 21' handeln. Diese braucht in der Regel nur einmal je tragbare Gasanalysevorrichtung 1 durchgeführt zu werden. Eine Ausnahme hiervon ist eine Reparatur 19, insbesondere eine umfangreiche Reparatur, die als ein Wiederherstellen angesehen wird; in diesem Fall wird die Werkseinstellung bzw. Werkskalibrierung erneut durchgeführt. Ein zweiter Teil der Kalibrierung wird vom Anwender selbst durchgeführt, und zwar in Abhängigkeit von dem vorgesehenen Messprogramm. Die Durchführung durch den Anwender erfolgt vor Ort, womit gemeint ist, dass sowohl zeitlich wie auch räumlich eine möglichst enge Anbindung zwischen Anwender-Kalibrierung 31 der tragbaren Gasanalysevorrichtung 1 und deren Verwendung zum Messen 41 erreicht werden soll. Auf diese Weise können Fehler wegen sich verändernder, abweichender Umgebungsbedingungen minimiert bzw. praktisch ausgeschlossen werden.

Die sei nachfolgend anhand zweier Ausführungsbeispiele näher erläutert, wobei das erste Beispiel für eine einfachere Variante und das zweite Beispiel für eine aufwendigere Variante steht.

### Ausführungsbeispiel 1:

In einem ersten Ausführungsbeispiel ist ein Gaschromatograph als integriertes tragbares Gasmessgerät ausgeführt. Jedes tragbare Gasmessgerät wird nach der Produktion 11 durch den Hersteller kalibriert 21' und eine Auswahl von Programmen 40 zu verschiedenen Stoffen aufgespielt 12. Der Anwender führt zu Beginn jedes Messtages eine Kalibrierung 31 mit einem vom Hersteller gelieferten Gasstandard 9 in den jeweiligen Umweltbedingungen durch, wobei das Gasmessgerät ein Gaschromatogramm mit mehreren spezifischen Signalspitzen (=Peaks) aufzeichnet und als Kalibrierdaten 32 abspeichert. Nun kann der Anwender das tragbare Gasmessgerät für eine Reihe von Messungen im Feld benutzen, so lange die Kalibrierung gültig ist, sich also insbesondere die Umweltbedingungen nicht zu stark verändern oder kein vorschriftsmäßiger Zeitraum überschritten wird. Bei der Messung führt das tragbare Gasmessgerät das Messverfahren 41 mit den Parametern aus, die im ausgewählten Programm 40 gespeichert wurden, wobei die so gemessenen Messdaten 42 mit Werten aus der Werkskalibrierung 21 korrigiert werden. Bei der Analyse wird die Messkurve mit den Referenzwerten der Kalibrierung am Gasstandard in beiden Koordinaten (typischerweise die Intensität linear und die Retentionszeit mit einer Polynomfunktion) kalibriert 32. Ein in der Recheneinheit implementierter Auswertungsalgorithmus 50 simuliert nun ein Modell für die erwarteten Signalspitzen (=Peaks) der Zielstoffe, die im Programm 40 vorgegeben sind, und passt die Parameter für Stoffkonzentrationen des Modells iterativ an, bis Modell und kalibrierte Messkurve übereinstimmen oder das Verfahren erfolglos abgebrochen wird. Eine im Algorithmus umgesetzte sogenannte Gerätefunktion verbindet dabei Modell und Messwerte. Als Ergebnis zeigt das tragbare Gasmessgerät direkt die ermittelten Konzentrationen der identifizierten Verbindungen in der Probe an 51 und speichert sie optional in einer Messdatenbank 52.

Nach der abgeschlossenen einzelnen Messung kann durch den Anwender mit weiteren Messungen fortgefahren werden, sofern die Kalibrierung noch gültig ist. Zu diesem Zweck führt das tragbare Gasmessgerät einen Gültigkeitstest 39 für die Kalibrierung durch, bei dem geprüft wird, ob die aktuellen Kalibrierdaten 32 beispielsweise nicht mehr als zwölf Stunden alt sind. Ist die Kalibrierung nicht mehr gültig, muss vor der nächsten Messung 41 eine neue Kalibriermessung 31 durchgeführt werden. Vor der neuen Kalibriermessung führt das tragbare Gasmessgerät einen Funktionstest aus, bei dem ein weiterer Algorithmus aus der vorangegangenen Serie von Messungen und der vorangegangenen Kalibriermessung auf die zureichende Funktionsfähigkeit schließt. Besteht das tragbare Gasmessgerät diesen Funktionstest 29 nicht, empfiehlt oder erzwingt das tragbare Gasmessgerät eine Reparatur 19, nach der eventuell eine neue Werkskalibrierung für ausgetauschte Komponenten durch den Hersteller nötig wird. Es ist hervorzuheben, dass in der beschriebenen Variante des Verfahrens nur die Kalibriermessung am Gasstandard, die Programmwahl und die Ausführung der Messungen vom Anwender aktiv ausgeführt werden, während die Auswertung 50 und die Selbst-Tests 39, 29 automatisch und jeweils im Bruchteil einer Sekunde aus den Datenbanken 12, 22, 32, 42, 52 berechnet werden können.

### Ausführungsbeispiel 2:

In einem zweiten Ausführungsbeispiel ist das Gasmessgerät ein tragbarer Gaschromatograph zur Messung volatiler organischer Verbindungen. Der Hersteller testet nach der Produktion 11 die Funktion des tragbaren Gasmessgerätes und speichert Korrekturwerte 21, die bei einer Reihe von Kalibriermessungen an unterschiedlichen Proben ermittelt werden, in einer Datenbank im Gasmessgerät. Die Korrekturwerte durch den Hersteller sind als Durchschnittswerte ermittelte Faktoren, wobei dies beispielsweise Leistungskoeffizienten der Gebläse und die Retentions- und Respons-Koeffizienten für jeden einzelnen während der Kalibrierung durch den Hersteller gemessenen Stoff sind. Koeffizienten für weitere Stoffe werden extrapoliert. Ebenfalls durch den Hersteller wird eine Datenbank mit verschiedenen Stoffen und den charakteristischen Peaks in deren Chromatogrammen auf das Gasmessgerät aufgespielt 12, die auch einen experimentell ermittelten Faktor enthalten, der die Signalhöhe an der verwendeten Detektoreinheit in eine Stoffkonzentration umrechnet. In der Kalibrierung durch den Anwender an einem Gasstandard 9 wird ein Chromatogramm für diesen Gasstandard 9 in den Umweltbedingungen der Messung gespeichert 32. Diese Referenzmessung wird verwendet, um das Chromatogramm der Messung bezüglich beider Dimensionen zu kalibrieren. Vor der Messung wird dabei ein Programm ausgewählt 40, das einerseits die Parameter der Messung und andererseits das Modell und die Startwerte der Analyse bestimmt. Der Anwender muss jeweils nur ein Programm wählen, zunächst das Programm zur Kalibrierung und dann das Programm des Zielstoffes, und die Messungen auslösen 41. Kalibrierung und Analyse inklusive aller Korrekturen werden automatisch durchgeführt, wobei als Ergebnis die Zusammensetzung und Konzentration der Stoffe angezeigt werden. Die Kalibriermessung muss dabei nicht Teil jeder Messung sein, weil durch das Auslassen der Kalibrierung der Vorrat des Gasstandards eingespart werden kann und entsprechend Kosten und Gewicht für das Mitführen des Gasstandards im Feldversuch gespart werden. Wie in Fig. 4 schematisch dargestellt, überlagern sich die Kalibrierdaten 32 und Messdaten 42 in einer gemeinsamen mehrdimensionalen Messkurve. Mit der Ausnahme von Problem- und Sonderfällen ist es für einen gut programmierten Algorithmus anhand der Position von Signalspitzen (=Peaks) möglich, die Bestandteile des Signals zuzuordnen und die kombinierte Messung in ihre Bestandteile zu trennen. Bei der Auswertung 50 werden in diesem zweiten Ausführungsbeispiel zusätzliche Daten herangezogen, nämlich eine Datenbank mit vorherigen Messungen 72, die mit dem gleichen Messprogramm 40 durchgeführt wurden, und ein Messwert eines Hilfsdetektors am Gasmessgerät, beispielsweise eines Thermometers, das die Außentemperatur aufzeichnet. Vorteilhaft wird diese Datenbank mit zusätzlichen Messdaten aus einer externen Datenbank 71 ergänzt. Weitere vorteilhafte Hilfsdaten sind beispielsweise die Seriennummer des Gasmessgerätes, der Zeitpunkt der Messung und die GPS-Koordinaten der Messung, Informationen die es erlauben, mehrere Messungen in Verbindung zu setzen. Aus den verfügbaren Daten kann die Analyse-Automatik innere und äußere Veränderungen für das spezielle Gasmessgerät an einem bestimmten Ort und dessen Abweichung von der Produktserie in den Algorithmen berücksichtigen und sogar vorteilhafte Anpassungen an der Programmwahl errechnen, die dem Anwender vorgeschlagen oder sogar automatisch umgesetzt werden. Durch die iterative Anpassung des Programms wird die Werkskalibrierung (im Vergleich zum ersten Ausführungsbeispiel) ersetzt, da das Gasmessgerät seine optimalen Parameter selbst findet. Auch können graduelle Verschlechterungen an Komponenten in begrenztem Rahmen durch das Programm kompensiert werden, um die Selbst-Tests 39, 29 länger zu bestehen (also auch tatsächlich in den dort vorgegeben Grenzen zu funktionieren), wodurch zusätzliche Kalibrierungen und/oder Reparaturen vermieden werden. In dieser Variante wird durch den Anwender aktiv nur ein Programm gewählt, dessen Feineinstellungen von der Automatik iterativ angepasst werden können, und ohne große Vorbereitung die Messung an der Probe ausführt. Damit bestehen für den Anwender ein bisher unerreichter Komfort und eine hohe Geschwindigkeit in der Ausführung von Messungen. Dagegen bestehen höhere Anforderungen an die Automatiken und die Toleranzen bei der Herstellung.

Zur Veranschaulichung der Definition einer mehrdimensionalen Messgröße ist in Fig. 4 ein beispielhaftes Messsignal einer Messung des Signals I(t) abgebildet, wobei I die Signalstärke und t die zweite Dimension der Messung ist. Hierbei sei anzumerken, dass t nicht der Zeitpunkt der Messung ist, also I(T) wie bei einer eindimensionalen Messung, sondern I(t,T) gilt. In der Gaschromatographie haben die Messsignale typischerweise die Form von mehreren, eventuell überlagerten Spitzen (=Peaks) mit eventuell asymmetrischen Flanken, die als Signale von Zielstoffen (X, Y, Z) und Referenzstoffen (A, B) in einer oder aufeinanderfolgenden Messungen aufgenommen werden. Bei einem Gaschromatographen mit Photoionisationsdetektor ist I das Signal an der Detektoreinheit und t die Retentionszeit im Gaschromatographen. Es wird typischerweise zuerst eine Gerätefunktion auf die Rohdaten der Messung angewandt, bevor diese in einer verständlichen Form dargestellt wird und ausgewertet werden kann. Die Methodik und mathematische Umsetzung der Analysealgorithmen in allen genannten Fällen ist herkömmlicherweise eine händische Zuordnung der Peaks zu Erfahrungswerten oder vorteilhaft eine automatisierte Simulation von Peaks (beispielsweise als modifizierte Gauß-Verteilung oder verwandte Funktionen), die über ein iteratives Verfahren an die Messkurve angelegt werden und weiter vorteilhaft automatisch den Erfahrungs- oder Theoriewerten von Zielstoffen zugeordnet werden. Das dargestellte Signal in Fig. 4 ist ein einfacher Fall mit klar definierten und trennbaren Peaks, bei dem eine händische, ggf. computergestützte Auswertung problemlos möglich ist. Auch einfache vollautomatische Analysealgorithmen ohne Korrekturen hätten mit diesem Beispiel keine Probleme. Kommt es dagegen zu Überlappungen, wird die Messung stark durch Hintergrundsignale und Rauschen gestört, oder befinden sich einige Peaks gerade so an der Grenze des Messbaren, dann wird ein aufwendiges und standardisierendes Verfahren zu Kalibrierung, Messung und Auswertung nötig, um eine verlässliche Zuordnung der Signalbestandteile zu ermöglichen. Eine besondere Herausforderung stellen sogenannte Querempfindlichkeiten dar, bei denen zwei Zielstoffe sehr ähnliche Signalcharakteristiken aufweisen, die sich überlagern, oder sogar die Messung nicht unabhängig ist, weil sich die Zielstoffe gegenseitig im Messprozess bzw. das Gasmessgerät beeinflussen.

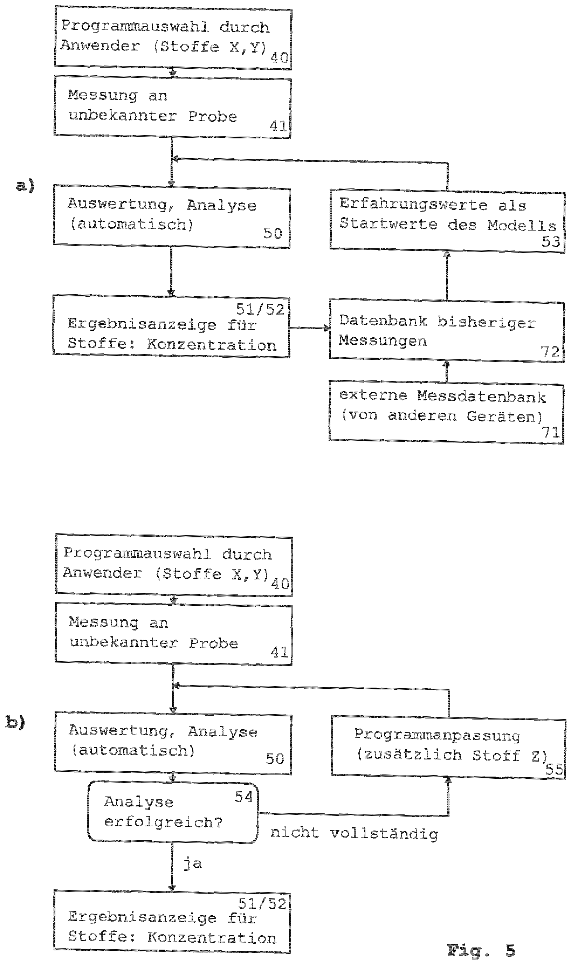

In Fig. 5 sind zwei Details des Verfahrens dargestellt, mit denen die Qualität der Analyse verbessert werden kann. In dem in Fig. 5a dargestellten Verfahren wird ein Messprogramm für zwei Zielstoffe (X, Y) gewählt und die Messung an einer unbekannten Probe durchgeführt. Das automatisierte Analyseprogramm ermittelt als Ergebnis die Konzentrationswerte für diese Stoffe und speichert die Messungen (nicht nur die Ergebnisse, sondern auch weitere Zwischenwerte oder sogar die Rohdaten) in einer Datenbank, die von einer externen Datenbank mit den Messungen, die mit demselben Programm auf anderen Gasmessgeräten durchgeführt wurden, zusammengeführt werden. Aus diesen Daten können nun Erfahrungswerte 53 berechnet werden, die sich insbesondere als Startwerte des Modells für eine Simulation bei weiteren Messungen verwenden lassen. Auch Grenzen der Simulation oder die Anpassung von solchen Parametern am Modell, die in der Simulation nicht variiert werden, können so iterativ verbessert werden. Auf diese Weise wird das Modell verbessert, das Gasmessgerät lernt automatisch. Auch können durch automatische Anpassungen am Modell Fehler aufgefangen werden und Wiederholungen der Messung vermieden werden. Im in Fig. 5b dargestellten Verfahren wird nach der Messung an einer unbekannten Probe mit einem Programm für zwei Zielstoffe (X, Y) getestet, ob die Analyse erfolgreich war 54 (beispielsweise ob die simulierten Peaks für X und Y sich mit dem gesamten Signal mit begrenzter Differenz überlagern) oder ob Signalteile nicht zugeordnet werden können. Wird die Analyse als "nicht erfolgreich" bewertet, wird in einer Programmschleife 55 die Analyse wiederholt mit zusätzlichen Zielstoffen aufgerufen, bis ein weiterer Zielstoff (Z) (bzw. ein anderes Programm) gefunden ist, der die Bedingung für eine erfolgreiche Analyse erfüllt (oder eine andere Austrittsbedingung die Schleife beendet).

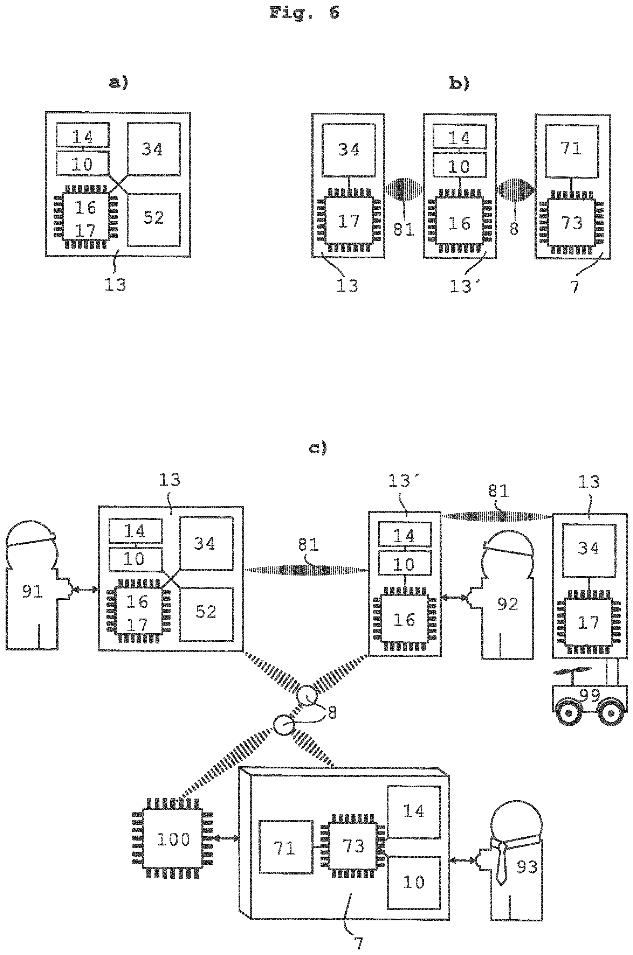

In Fig. 6 sind mehrere Varianten dargestellt, wie die verschiedenen physischen Komponenten des Messgerätes in Verbindung stehen. Wie in Fig. 6a dargestellt kann das Gasmessgerät 1 als integriertes Gasmessgerät mit Detektoreinheit 34, Steuer- und Auswerteeinheit 16/17 und interner Datenbank 52 ausgeführt sein und eine eigene Bedien- 10 und Anzeigeeinheit 14 haben. In einer anderen (von der Anmelderin umgesetzten) Variante ist das Gasmessgerät zweiteilig ausgeführt, als ein Gasmessgerät 1' mit Detektoreinheit 34 und Steuereinheit 17 und ein zweites abgesetztes Gerät 13', die zusammen drahtlos über Bluetooth verbunden sind, wobei das abgesetzte Gerät 13' konkret ein Smartphone mit Touchscreen 10, 14 ist und das Smartphone weiterhin über WLAN und/oder eine mobile Internetverbindung mit einer externen Recheneinheit, einem Server 7, verbunden ist, der eine eigene Recheneinheit 73 und eine externe Datenbank 71 besitzt. In einer dritten Variante sind die beiden vorherigen Varianten in einem funktionalen Verbund von mehreren Gasmessgeräten und mehreren Anwendern 91, 92, 93, wobei der Server als Steuerzentrale mit eigenen Bedien- und Anzeigeelementen 10, 14 ausgeführt wird und zusätzlich ein leistungsfähiger Computer 100 einen Teil der Steuerung der Operation übernimmt. In einer vorteilhaften Ausführung, ebenfalls in Fig. 6c dargestellt, ist ein Gasmessgerät, das von einem Anwender 92 mit dem abgesetzten Gerät 13' gesteuert wird, auf einer Trägereinheit 99, im Beispiel einer fahrenden und flugfähigen Drohne, aufgesetzt, die vorteilhaft selbstgesteuert ausgeführt ist. Dieses drohnengetragene Gasmessgerät kann an schwer zugängliche Orte gelangen und in besonderen Gefahrensituationen, insbesondere der Suche nach hochgiftigen und/oder explosiven Zielstoffen, eingesetzt werden.

## Patentansprüche

1. Verfahren zum mehrstufigen Kalibrieren von Mess- und Analyseverfahren für ein tragbares Gasmessgerät (1, 1') mit einer Detektoreinheit (34) für die Gaschromatographie zur selektiven Detektion einer Mehrzahl von chemischen Verbindungen, mit verbundener Steuereinheit (17) und mit verbundener Auswerteeinheit (16),
wobei in einem ersten Schritt (21) nach der Herstellung eine Werkseinstellung des tragbaren Gasmessgeräts (1, 1') und/oder einem dem tragbaren Gasmessgerät zugeordneten Geräts (13') vorgenommen wird, wobei eine Datenbank zu einer Mehrzahl von Zielstoffen und/oder Messprogrammen für das jeweilige tragbare Gasmessgerät generiert wird,
in einem zweiten Schritt durch den Anwender ein Behälter mit einem bekannten Gasstandard (91) an das tragbare Gasmessgerät angeschlossen (31) wird und an diesem Gasstandard eine Referenzmessung durchgeführt (32) wird, bei der eine mehrdimensionale Messgröße, insbesondere ein Gaschromatogramm, aufgezeichnet wird,
in einem dritten Schritt durch den Anwender ein Messprogramm für einen oder mehrere Zielstoffe aus einer Liste von Messprogrammen ausgewählt (40) wird, wobei die Messprogramme durch den Hersteller auf das Gerät aufgespielt (12) wurden oder durch den Anwender erstellt sein können,
in einem vierten Schritt eine Messung ausgelöst (41) wird, wobei die Steuereinheit das Messverfahren in Abhängigkeit des gewählten Messprogramms ausführt, bei dem eine mehrdimensionale Messgröße aufgezeichnet (42) wird,
und in einem fünften Schritt die Auswerteeinheit automatisiert eine Analyse (50) zur Identifizierung der in einer Probe vorhandenen Zielstoffe und deren jeweilige Konzentrationen durchführt, wobei ein Algorithmus mehrere Eingabegrößen verarbeitet, darunter die mehrdimensionale Messgröße aus der Messung (42) an der Probe, die mehrdimensionale Referenzmessung am Gasstandard, den Werten für den Zielstoff oder die Zielstoffe für das jeweilige Programm und die Werkeinstellung für das tragbare Gasmessgerät.

2. Verfahren zum mehrstufigen Kalibrieren von Mess- und Analyseverfahren für ein tragbares Gasmessgerät (1, 1') mit einer Detektoreinheit (34) für die Gaschromatographie zur selektiven Detektion einer Mehrzahl von chemischen Verbindungen, mit verbundener Steuereinheit (17) und mit verbundener Auswerteeinheit (16),
wobei in einem ersten Schritt (21) nach Herstellung eine Werkseinstellung des tragbaren Gasmessgeräts (1, 1') und/oder einem dem tragbaren Gasmessgerät zugeordneten Geräts (13') vorgenommen wird, wobei eine Datenbank zu einer Mehrzahl von Zielstoffen und/oder Messprogrammen für das jeweilige tragbare Gasmessgerät generiert wird,
in einem zweiten Schritt durch den Anwender ein Messprogramm für einen oder mehrere Zielstoffe aus einer Liste von Messprogrammen ausgewählt wird, wobei die Messprogramme entweder durch den Hersteller auf das Gerät aufgespielt wurden oder durch den Anwender erstellt sein können,
in einem dritten Schritt eine Messung ausgelöst wird, wobei die Steuereinheit das Messverfahren in Abhängigkeit des gewählten Messprogramms ausführt, bei dem eine mehrdimensionale Messgröße, insbesondere ein Gaschromatogramm, aufgezeichnet wird, in der das Signal einer Probe mit dem Signal einer Referenzmessung überlagert ist, wobei als Referenz ein bekannter Gasstandard verwendet wird, der im oder am Gerät vorgehalten wird und im Gerät mit der Probe vermischt wird,
und in einem vierten Schritt die Auswerteeinheit automatisiert eine Analyse zur Identifizierung der in der Probe vorhandenen Zielstoffe und deren jeweilige Konzentrationen durchführt,
wobei ein Algorithmus die Signale von Probe und Referenz aus der mehrdimensionalen Messgröße trennt und diese zusammen mit weiteren Eingabegrößen aus Datenbanken verarbeitet, darunter die Werte der Zielstoffe für das jeweilige Programm und die Werkseinstellung für das Gerät.

3. Verfahren nach einem der vorangehenden Ansprüche, wobei durch den Hersteller eine Werkskalibrierung (21) durchgeführt wird, bei der für das jeweilige tragbare Gasmessgerät (1, 1') eigene Gerätekorrekturwerte ermittelt (22) und in einer Datenbank gespeichert werden.

4. Verfahren nach einem der vorangehenden Ansprüche, wobei es sich bei dem Gasmessgerät (1, 1') um einen Gaschromatographen handelt, der als miniaturisiertes, tragbares Messgerät für Feldversuche unter 1,5 kg Gesamtgewicht ausgeführt ist.

5. Verfahren nach einem der vorangehenden Ansprüche, wobei der Gasstandard ein Gasgemisch mit einem oder mehreren ungefährlichen Stoffen ist, insbesondere alle enthaltenen Stoffe im Gasstandard nicht gesundheitsschädlich sind oder in ungefährlicher Konzentration vorliegen, wogegen einer oder mehrere vermutete Zielstoffe in der Probe gesundheitsschädliche Stoffe sind, also möglicherweise in einer gefährlichen Konzentration aufgefunden werden.

6. Verfahren nach einem der vorangehenden Ansprüche, wobei als Gasstandard (91) der Kalibriermessung (31) ein Gasgemisch der Stoffe Isobuten (A) und Toluol (B) in jeweils ungefährlicher Konzentration, insbesondere jeweiligen Konzentrationen zwischen 1 ppm und 100 ppm in Luft oder Stickstoff, verwendet wird und wobei die Zielstoffe des Messprogramms (40) der Probenmessung (41) die bereits in geringer Konzentration schädlichen Stoffe Benzol (X) und 1,3-Butadien (Y) sind, deren Detektionsgrenze jeweils unter 1 ppm, vorteilhaft sogar unter 1 ppb, liegt, und wobei in der Analyse auch Signalanteile weiterer unbekannter Stoffe (Z) im Messsignal von den Signalanteilen der Zielstoffe (X, Y) unterschieden werden können

7. Verfahren nach einem der vorangehenden Ansprüche, wobei das tragbare Gasmessgerät (1, 1') mit einer mobilen Recheneinheit ausgestattet oder verbunden ist, die über eine drahtlose Verbindung auf Datenbanken von externen Recheneinheiten zugreifen kann,
wobei vorzugsweise durch das Gerät zusätzlich Daten aus externen Datenbanken abgerufen werden, die durch den Algorithmus der Analyse verarbeitet werden.

8. Verfahren nach einem der vorangehenden Ansprüche, wobei das tragbare Gasmessgerät (1, 1') die mehrdimensionale Messgröße aufeinanderfolgender Messungen in einer Datenbank speichert und dabei über einen Algorithmus aus den Messergebnissen der Referenzprobe einen Funktionstest des tragbaren Gasmessgerätes (1, 1') durchführt, nachdem dem Anwender Informationen über den Zustand des tragbaren Gasmessgerätes (1, 1'), insbesondere eine Warnung über eine nötige Wartung, anzeigt werden.

9. Verfahren nach einem der vorangehenden Ansprüche, wobei das tragbare Gasmessgerät (1, 1') vorangegangene Messergebnisse, die ebenfalls mit dem gewählten Programm erzeugt wurden, aus einer internen Datenbank in den Algorithmus der Analyse einbezieht.

10. Verfahren nach einem der vorangehenden Ansprüche, wobei das tragbare Gasmessgerät (1, 1') Messergebnisse, die von anderen solchen tragbaren Gasmessgeräten des gleichen Typs (1, 1') ebenfalls mit dem gewählten Programm erzeugt wurden, aus einer internen oder externen Datenbank in den Algorithmus der Analyse einbezieht.

11. Verfahren nach einem der vorangehenden Ansprüche, wobei das Messverfahren automatisiert an die Messergebnisse von Hilfsdetektoren, insbesondere der Außentemperatur, angepasst wird,
und/oder
das tragbare Gasmessgerät (1, 1') dem Anwender automatisiert Änderungen am Messprogramm vorschlägt, die es aus den in Datenbanken gespeicherten vorangegangenen Messungen in einem Algorithmus als mit Wahrscheinlichkeit geeignetere Parameter berechnet hat.

12. Verfahren nach einem der vorangehenden Ansprüche, wobei der Algorithmus der Analyse, nachdem ein vorangegangener Durchgang der Analyse nach bestimmten Kriterien als fehlgeschlagen bewertet wird, automatisiert nachträglich weitere Zielstoffe aus einer Datenbank in das Programm der Analyse hinzufügt (55), die das durch den Anwender vorgewählte Messprogramm ergänzen, wobei dieser Vorgang wiederholt wird, bis entweder die Analyse erfolgreich wird oder eine bestimmte Zahl von Wiederholungen bzw. Zeitspanne überschritten wird.

13. Verfahren nach einem der vorangehenden Ansprüche, wobei für ein Messprogramm (40) mehrerer Zielstoffe oder der Auswahl einer Gruppe von Zielstoffen aus einer Liste von Stoffen das Steuerprogramm der Messung und/oder der Algorithmus der Analyse an der Auswerteeinheit (16, 73) automatisch so angepasst wird, dass eine bekannte Querempfindlichkeit für diese Zielstoffe vermieden wird.

14. Verfahren nach einem der vorangehenden Ansprüche, wobei die Messung parallel von mehreren Anwendern (91, 92) durchgeführt wird, und während des Messeinsatzes die Einstellung der Messprogramme und/oder Daten zwischen mehreren in Netzwerken (8, 81) verbundenen tragbaren Gasmessgeräten (1, 1') und/oder zu einem zentralen Server (7) übermittelt werden.

15. Verfahren nach einem der vorangehenden Ansprüche, wobei das tragbare Gasmessgerät als Teil oder Aufsatz an einem durch den Anwender ferngesteuerten und/oder selbstgesteuerten Fahrzeug und/oder Fluggerät, insbesondere einer Roboter-Drohne, als Trägereinheit (99) verwendet wird und das Gasmessgerät über eine drahtlose Verbindung (8, 81) an eine externe Recheneinheit (16, 73) verbunden wird.

## Claims

1. Method for the multistep calibration of measuring and analysis methods for a portable gas measuring device (1, 1') having a detector unit (34) for gas chromatography for selective detection of a plurality of chemical compounds, having a connected control unit (17) and having a connected analysis unit (16),
wherein, in a first step (21) after production, a factory setting of the portable gas measuring device (1, 1') and/or a device (13') associated with the portable gas measuring device is performed, wherein a database is generated for a plurality of target materials and/or measuring programs for the respective portable gas measuring device,
in a second step, a container having a known gas standard (91) is connected (31) to the portable gas measuring device by the user and a reference measurement is performed (32) on this gas standard, in which a multidimensional measured variable is recorded, in particular a gas chromatogram,
in a third step, a measuring program for one or more target materials is selected (40) by the user from a list of measuring programs, wherein the measuring programs were installed (12) on the device by the producer or can be prepared by the user,
in a fourth step, a measurement is triggered (41), wherein the control unit executes the measuring method in dependence on the selected measuring program, in which a multidimensional measured variable is recorded (42),
and, in a fifth step, the analysis unit automatically carries out an analysis (50) to identify the target materials present in a sample and the respective concentrations thereof, wherein an algorithm processes multiple input variables, among them the multidimensional measured variable from the measurement (42) on the sample, the multidimensional reference measurement on the gas standard, the values for the target material or the target materials for the respective program, and the factory setting for the portable gas measuring device.

2. Method for the multistep calibration of measuring and analysis methods for a portable gas measuring device (1, 1') having a detector unit (34) for gas chromatography for selective detection of a plurality of chemical compounds, having a connected control unit (17) and having a connected analysis unit (16),
wherein, in a first step (21) after production, a factory setting of the portable gas measuring device (1, 1') and/or a device (13') associated with the portable gas measuring device is performed, wherein a database is generated for a plurality of target materials and/or measuring programs for the respective portable gas measuring device,
in a second step, a measuring program for one or more target materials is selected by the user from a list of measuring programs, wherein the measuring programs were either installed on the device by the producer or can be prepared by the user,
in a third step, a measurement is triggered, wherein the control unit executes the measuring method in dependence on the selected measuring program, in which a multidimensional measured variable, in particular a gas chromatogram, is recorded, in which the signal of a sample is superimposed with the signal of a reference measurement, wherein a known gas standard, which is kept ready in or on the device and is mixed with the sample in the device, is used as the reference,
and, in a fourth step, the analysis unit automatically carries out an analysis to identify the target materials present in the sample and the respective concentrations thereof,
wherein an algorithm separates the signals of the sample and the reference out of the multidimensional measured variable and processes them together with further input variables from databases, among them the values of the target materials for the respective program and the factory setting for the device.

3. Method according to any one of the preceding claims, wherein a factory calibration (21) is performed by the producer, in which separate device correction values are determined (22) for the respective portable gas measuring device (1, 1') and stored in a database.

4. Method according to any one of the preceding claims, wherein the gas measuring device (1, 1') is a gas chromatograph, which is embodied as a miniaturized, portable measuring device for field tests with a total weight of less than 1.5 kg.

5. Method according to any one of the preceding claims, wherein the gas standard is a gas mixture having one or more harmless materials, in particular all materials contained in the gas standard are not harmful to health or are present in harmless concentrations, while in contrast one or more suspected target materials in the sample are materials hazardous to health, i.e., are possibly found in a hazardous concentration.

6. Method according to any one of the preceding claims, wherein a gas mixture of the materials isobutene (A) and toluene (B), each in a harmless concentration, in particular respective concentrations between 1 ppm and 100 ppm in air or nitrogen, is used as the gas standard (91) of the calibration measurement (31), and wherein the target materials of the measuring program (40) of the sample measurement (41) are the materials benzene (X) and 1,3-butadiene (Y), which are already harmful at low concentrations and the detection limit of which is less than 1 ppm, advantageously even less than 1 ppb in each case, and wherein signal components of further unknown materials (Z) in the measurement signal can also be differentiated from the signal components of the target materials (X, Y) in the analysis.

7. Method according to any one of the preceding claims, wherein the portable gas measuring device (1, 1') is equipped with or connected to a mobile processing unit, which can access databases of external processing units via a wireless connection,
wherein data, which are processed by the algorithm of the analysis, are preferably additionally retrieved from external databases by the device.

8. Method according to any one of the preceding claims, wherein the portable gas measuring device (1, 1') stores the multidimensional measured variable of successive measurements in a database and carries out a function test of the portable gas measuring device (1, 1') via an algorithm from the measurement results of the reference sample, after which items of information about the state of the portable gas measuring device (1, 1'), in particular a warning about required maintenance, are displayed to the user.

9. Method according to any one of the preceding claims, wherein the portable gas measuring device (1, 1') incorporates prior measurement results, which were also generated using the selected program, from an internal database in the algorithm of the analysis.

10. Method according to any one of the preceding claims, wherein the portable gas measuring device (1, 1') incorporates measurement results, which were also generated using the selected program by other such portable gas measuring devices of the same type (1, 1'), from an internal or external database in the algorithm of the analysis.

11. Method according to any one of the preceding claims, wherein the measuring method is automatically adapted to the measurement results of auxiliary detectors, in particular the external temperature,
and/or
the portable gas measuring device (1, 1') automatically proposes changes to the measuring program to the user, which it has computed in an algorithm from the prior measurements stored in databases as parameters which are probably more suitable.

12. Method according to any one of the preceding claims, wherein the algorithm of the analysis, after a preceding pass of the analysis has been evaluated as failed according to specific criteria, subsequently automatically adds (55) further target materials from a database into the program of the analysis, which supplement the measuring program preselected by the user, wherein this procedure is repeated until either the analysis becomes successful or a specific number of repetitions and/or a time span is exceeded.

13. Method according to any one of the preceding claims, wherein, for a measuring program (40) of multiple target materials or the selection of a group of target materials from a list of materials, the control program of the measurement and/or the algorithm of the analysis is/are automatically adapted to the analysis unit (16, 73) so that a known cross-sensitivity for these target materials is avoided.

14. Method according to any one of the preceding claims, wherein the measurement is carried out in parallel by multiple users (91, 92), and, during the measurement use, the setting of the measuring programs and/or data is/are transferred between multiple portable gas measuring devices (1, 1') connected in networks (8, 81) and/or to a central server (7).

15. Method according to any one of the preceding claims, wherein the portable gas measuring device is used as a part or attachment on a vehicle and/or aircraft, in particular a robot drone, which is remote-controlled by the user and/or self-controlled, as a carrier unit (99), and the gas measuring device is connected via a wireless connection (8, 81) to an external processing unit (16, 73).

## Revendications

1. Procédé d'étalonnage en plusieurs étapes de procédés de mesure et d'analyse pour un appareil de mesure de gaz portable (1, 1') doté d'une unité de détection (34) pour la chromatographie en phase gazeuse servant à la détection sélective d'une pluralité de composés chimiques, comprenant une unité de commande reliée (17) et une unité d'évaluation reliée (16),
dans lequel, dans une première étape (21) après la fabrication, un réglage usine de l'appareil de mesure de gaz portable (1, 1') et/ou d'un appareil (13') associé à l'appareil de mesure de gaz portable est effectué, une base de données concernant une pluralité de substances cibles et/ou de programmes de mesure étant générée pour l'appareil de mesure de gaz portable respectif,
dans une deuxième étape, l'utilisateur connecte (31) un récipient avec un étalon de gaz connu (91) à l'appareil de mesure de gaz portable, et effectue (32) sur cet étalon de gaz une mesure de référence lors de laquelle une grandeur de mesure multidimensionnelle, en particulier un chromatogramme en phase gazeuse, est enregistrée,
dans une troisième étape, l'utilisateur sélectionne (40) un programme de mesure pour une ou plusieurs substances cibles sur une liste de programmes de mesure, les programmes de mesure ayant été installés (12) par le fabricant sur l'appareil ou pouvant être créés par l'utilisateur,
dans une quatrième étape, une mesure est déclenchée (41), l'unité de commande exécutant le procédé de mesure en fonction du programme de mesure sélectionné dans lequel une grandeur de mesure multidimensionnelle est enregistrée (42),
et dans une cinquième étape, l'unité d'évaluation effectue de manière automatisée une analyse (50) pour identifier les substances cibles présentes dans un échantillon et leurs concentrations respectives, un algorithme traitant plusieurs grandeurs d'entrée, parmi lesquelles se trouvent la grandeur de mesure multidimensionnelle issue de la mesure (42) sur l'échantillon, la mesure de référence multidimensionnelle sur l'étalon de gaz, les valeurs pour la substance cible ou les substances cibles pour le programme respectif et le réglage usine pour l'appareil de mesure de gaz portable.

2. Procédé d'étalonnage en plusieurs étapes de procédés de mesure et d'analyse pour un appareil de mesure de gaz portable (1, 1') doté d'une unité de détection (34) pour la chromatographie en phase gazeuse servant à la détection sélective d'une pluralité de composés chimiques, comprenant une unité de commande reliée (17) et une unité d'évaluation reliée (16),
dans lequel, dans une première étape (21) après la fabrication, un réglage usine de l'appareil de mesure de gaz portable (1, 1') et/ou d'un appareil (13') associé à l'appareil de mesure de gaz portable est effectué, une base de données concernant une pluralité de substances cibles et/ou de programmes de mesure étant générée pour l'appareil de mesure de gaz portable respectif,
dans une deuxième étape, l'utilisateur sélectionne un programme de mesure pour une ou plusieurs substances cibles sur une liste de programmes de mesure, les programmes de mesure ayant été installés sur l'appareil par le fabricant ou pouvant être créés par l'utilisateur,
dans une troisième étape, une mesure est déclenchée, l'unité de commande exécutant le procédé de mesure en fonction du programme de mesure sélectionné dans lequel une grandeur de mesure multidimensionnelle, en particulier un chromatogramme en phase gazeuse, est enregistrée dans laquelle le signal d'un échantillon est superposé à au signal d'une mesure de référence, un étalon de gaz connu étant utilisé comme référence qui est fourni dans ou au niveau de l'appareil et qui est mélangé avec l'échantillon dans l'appareil,
et dans une quatrième étape, l'unité d'évaluation effectue de manière automatisée une analyse pour identifier les substances cibles présentes dans l'échantillon et leurs concentrations respectives,
un algorithme séparant les signaux de l'échantillon et de la référence à partir de la grandeur de mesure multidimensionnelle et les traite avec d'autres grandeurs d'entrée provenant de bases de données, parmi lesquelles se trouvent les valeurs des substances cibles pour le programme respectif et le réglage usine pour l'appareil.

3. Procédé selon l'une quelconque des revendications précédentes, dans lequel le fabricant effectue un étalonnage d'usine (21) dans lequel des valeurs de correction d'appareil propres sont établies (22) pour l'appareil de mesure de gaz portable (1, 1') respectif et sont sauvegardées dans une base de données.

4. Procédé selon l'une quelconque des revendications précédentes, dans lequel l'appareil de mesure de gaz (1, 1') est un chromatographe en phase gazeuse qui est réalisé sous la forme d'un appareil de mesure portable miniaturisé destiné à des essais de terrain avec un poids total inférieur à 1,5 kg.

5. Procédé selon l'une quelconque des revendications précédentes, dans lequel l'étalon de gaz est un mélange de gaz avec une ou plusieurs substances non dangereuses, en particulier aucune des substances contenues dans l'étalon de gaz n'est nocive ou est présente en concentration non dangereuse, alors qu'une ou plusieurs substances cibles supposées dans l'échantillon sont des substances nocives, c'est-à-dire qu'elles pourraient s'y trouver en concentration dangereuse.

6. Procédé selon l'une quelconque des revendications précédentes, dans lequel comme étalon de gaz (91) de la mesure d'étalonnage (31), un mélange de gaz des substances isobutène (A) et toluol (B) respectivement en concentration non dangereuse, en particulier dans des concentrations respectives comprises entre 1 ppm et 100 ppm dans l'air ou l'azote, est utilisé, et dans lequel les substances cibles du programme de mesure (40) de la mesure d'échantillon (41) sont les substances benzène (X) et 1,3-butadiène (Y), nocives même en faible concentration, dont la limite de détection se situe respectivement au-dessous de 1 ppm, avantageusement même au-dessous de 1 ppb, et dans lequel dans l'analyse, il est également possible de distinguer des parts de signal d'autres substances inconnues (Z) dans le signal de mesure par rapport aux parts de signal des substances cibles (X, Y).

7. Procédé selon l'une quelconque des revendications précédentes, dans lequel l'appareil de mesure de gaz portable (1, 1') est équipé de ou relié à une unité de calcul mobile qui peut accéder à des bases de données d'unités de calcul externes par l'intermédiaire d'une liaison sans fil,
l'appareil extrayant de préférence en outre des données provenant de bases de données externes qui sont traitées par l'algorithme de l'analyse.

8. Procédé selon l'une quelconque des revendications précédentes, dans lequel l'appareil de mesure de gaz portable (1, 1') mémorise la grandeur de mesure multidimensionnelle de mesures consécutives dans une base de données et effectue alors par un algorithme à partir des résultats de mesure de l'échantillon de référence un test fonctionnel de l'appareil de mesure de gaz portable (1, 1'), après que l'utilisateur a reçu un affichage d'informations concernant l'état de l'appareil de mesure de gaz portable (1, 1'), en particulier un avertissement concernant une maintenance nécessaire.

9. Procédé selon l'une quelconque des revendications précédentes, dans lequel l'appareil de mesure de gaz portable (1, 1') intègre dans l'algorithme de l'analyse des résultats de mesure antérieurs qui ont également été générés par le programme sélectionné et proviennent d'une base de données interne.

10. Procédé selon l'une quelconque des revendications précédentes, dans lequel l'appareil de mesure de gaz portable (1, 1') intègre dans l'algorithme de l'analyse les résultats de mesure qui ont été produits par d'autres de ces appareils de mesure de gaz portables du même type (1, 1') également avec le programme sélectionné à partir d'une base de données interne ou externe.

11. Procédé selon l'une quelconque des revendications précédentes, dans lequel le procédé de mesure est adapté de manière automatisée aux résultats de mesure de détecteurs auxiliaires, en particulier de la température extérieure, et/ou
l'appareil de mesure de gaz portable (1, 1') propose de manière automatisée à l'utilisateur des changements du programme de mesure qu'il a calculés à partir des mesures précédentes, mémorisées dans les bases de données, dans un algorithme comme des paramètres probablement mieux appropriés.

12. Procédé selon l'une quelconque des revendications précédentes, dans lequel l'algorithme de l'analyse, après qu'un passage précédent de l'analyse a été évalué comme ayant échoué selon certains critères, ajoute (55) au programme de l'analyse de manière automatisée a posteriori d'autres substances cibles provenant d'une base de données qui complètent le programme de mesure présélectionné par l'utilisateur, ce processus étant répété jusqu'à ce que soit l'analyse réussisse, soit un certain nombre de répétitions ou une période de temps soit dépassé (e) .

13. Procédé selon l'une quelconque des revendications précédentes, dans lequel pour un programme de mesure (40) de plusieurs substances cibles ou de la sélection d'un groupe de substances cibles sur une liste de substances, le programme de commande de la mesure et/ou l'algorithme de l'analyse au niveau de l'unité d'évaluation (16, 73) est/sont adapté(s) automatiquement de façon à éviter une sensibilité croisée connue pour ces substances cibles.

14. Procédé selon l'une quelconque des revendications précédentes, dans lequel la mesure est effectuée en parallèle par plusieurs utilisateurs (91, 92), et pendant l'intervention de mesure, le réglage des programmes de mesure et/ou des données sont transmis entre plusieurs appareils de mesure de gaz portables (1, 1') reliés par des réseaux (8, 81) et/ou à un serveur central (7).

15. Procédé selon l'une quelconque des revendications précédentes, dans lequel l'appareil de mesure de gaz portable est utilisé comme une partie ou un accessoire d'un véhicule et/ou engin volant, en particulier d'un drone robotisé, télécommandé(s) par l'utilisateur et/ou autonome(s), comme unité de support (99), et l'appareil de mesure de gaz est relié à une unité de calcul externe (16, 73) par une liaison sans fil (8, 81).
